# EUROPEAN PATENT SPECIFICATION

(11) **EP 0 558 131 B1**
(45) Date of publication and mention of the grant of the patent: **15.04.1998**
(21) Application number: 93200475.7
(22) Date of filing: 19.02.1993
(51) Int. Cl.: G01N 25/20, G01N 25/16, G01N 33/22

(54) **Method for deciding the reactivity and soot index of carbon products and equipment therefor**
Verfahren und Vorrichtung zur Feststellung der Reaktivität und des Russindexes von Kohlenstoffprodukten
Procédé et appareil pour la détermination de la réactivité et de l'indice de suie des produits carboniques

(30) Priority: 28.02.1992 NO 920789
(43) Date of publication of application: 01.09.1993
(73) Proprietor: NORSK HYDRO ASA, 0240 Oslo (NO)
(72) Inventor: Bergli, Knut, N-3472 Bodalen (NO); Foosnaes, Trygve, N-5875 Ardalstangen (NO); Naterstad, Tormod, N-1370 Asker (NO)
(74) Representative: Bleukx, Luc

(56) References cited:
- EP-A- 0 327 914
- GB-A- 2 154 318
- US-A- 4 976 549

## Description

The present invention concerns a new method for deciding the reactivity and soot index of carbon products such as granular coke and burned carbon core samples, and equipment therefor.

Everyone involved in traditional aluminium production knows full well that some of the anode material takes part in reactions which do not benefit the production of the metal.

The most obvious aspect is the corrosion of the top of the anode where it comes into contact with the air. In a more concealed manner, mainly on the underside of the anode, another unfortunate process takes place. Here some of the CO₂ gas from the primary reaction reacts with the carbon of the anode to form CO, carbon monoxide. According to P.J. Rhedey, Alcan International Limited, Kingston Laboratories, in "Carbon reactivity and aluminium reduction cell anodes", the reaction with air and the reaction with CO₂ contribute to a significant part of the anode consumption.

The reaction with air and CO₂ can, furthermore, cause the anode material to crumble, which causes operational problems with anode particles in the electrolyte, so-called sooting.

From these facts it is not difficult to understand that, for both the anode producer and the user, it is necessary to be able to predict some of the anode's tendency to react with air and carbon dioxide.

"GB-A-2 154 318, discloses a method for assessing the micro-reactivity of coke, more specific the CO₂-reactivity of such material. This publication further discloses that the equipment involved may comprise a furnace, a device for weighing, a device for temperature registration and a device for the introduction of gas in the furnace. However, this kind of technique is limited to the determination of the CO₂-reactivity of the test material, and do not disclose analysis of air reactivity and soot index.

EP-A-0 327 914, discloses a calorimetry system for measurement of the heating value of solid fuel (coal), having a combustor and a mixing unit wherein heat from combustion gases is transferred to air. Instrumentation in the system measures the flow rates of cooling air, primary air which carries the coal streams into the combustor and secondary combustion air as well as the mass flow rates of the coal into the combustor as measured with a gravimetric feeder.

A computer is provided with input/output devices responsive to measuring instruments (thermocouples and pressure gauges) for controlling the feeding of the coal and fuel gases during initiation of combustion, and for computing the heating value of the coal.
The above mentioned system is designed for the determination of the heating value of solid fuels, and thus it represents an analysis that differs from the analysis in the present invention which relates to air-/CO₂ reactivity and soot index of a carbon product.

US-A-4 976 549, relates to a microdilatometer for direct measurement of coal ash sintering and fusing properties at elevated temperatures and pressures. The equipment comprises electrical resistivity measurements across a sample to provide a measurement of the onset of the sintering and fusion of the ash particulates while the contraction of the sample during sintering is measured with a linear variable displacement transducer for detecting the initiation of sintering. This apparatus is limited to the performance of dilatometry measurements, and thus relates to another type of analysis than defined by the present invention."

The reactivity of anodes can be measured in a number of ways, depending on how we choose to attack the problem. In general terms, regardless of the angle of attack, such a method must be at least:
- selective;
- sufficiently sensitive;
- sufficiently reproducible.

A selective method is a method which mainly reacts to the properties of the anode which are of importance for the air/CO₂ reaction under operating conditions.

Satisfactory sensitivity we will define as the ability to reveal changes in the stated properties at a level at which this would be of significance for the operation of the electrolysis cells.

The reproducibility, i.e. the distribution of the results from tests on several samples from the same anode, should not be so large that it is of real significance in the assessment of the anode's user properties. This is the same as saying that, regardless of how many times a piece of carbon is tested, the conclusion with regard to its quality must remain the same.

In the method of analysis which forms the object of the present invention, instead of scaling down the operating conditions in the electrolysis cells to manageable laboratory size, emphasis is laid on developing a reproducible test which can put us in a position to carry out quality grading of anodes. In addition, the point of departure for the grading is the hypothesis that the lowest possible reactivity and soothing are desirable.

The anode's tendency to react - i.e. how rapidly the reaction takes place - has been designated "reactivity". Thus we are here talking about two types of reactivity, namely CO₂ reactivity and air reactivity. Both reactions lead to a gasification of the sample, i.e. it loses weight. By registering this loss of weight we can get a measurement of the reactivity.

Sooting is caused in this connection by the reactivity being different in the aggregate and the binding agent. If the binding coke, for example, reacts least, this will cause the particles in the aggregate to be undermined and gradually to fall out during the test (and under operating conditions). After the sample has been exposed to the reactant gas over a certain period of time, it is brushed and all the loose material is collected and weighed. This is thus soot in the context of the analysis. We have chosen to express the level of sooting as the ratio of the weight of the soot and the overall loss in weight, where the overall loss of weight is the total weight of the gasified part and the part brushed off.

The reactivities are determined by means of a regression analysis for the last 30 minutes of the reaction time.

The following formulae apply:$\text{Reactivity =} \frac{{\text{G}}_{\text{30}}}{{\text{π*D*L*t}}_{\text{30}}} {\text{, mg.cm}}^{\text{-2}} {\text{.h}}^{\text{-1}}$$\text{Soot index =} \frac{\text{100*S}}{\text{S + G}} \text{, %}$
- G =: gasified (weight loss) during the whole test (max. 190 min.), mg.
- G₃₀ =: gasified (weight loss) during the last 30 min., mg.
- S =: soot formed with normal test length (max. 190 min.), mg.
- D =: sample diameter, cm.
- L =: sample length, cm.
- t₃₀ =: ½ hour.

In the test methods for deciding air and CO₂ reactivity which have been known up to now, at least two independent tests must be carried out; one to decide the air reactivity and another to decide the CO₂ reactivity.

The present invention concerns a method for deciding the air and CO₂ reactivity of a sample of a carbon product in one and the same test. The soot index can also be determined on the basis of this test.

This method is very time-saving in relation to existing techniques in which air and CO₂ reactivity have to be decided from separate tests. Time is saved both because it is only necessary to prepare and handle one sample to carry out an analysis of both air and CO₂ reactivity and because only one heating and cooling period is necessary. In addition, less manpower is required to carry out this analysis than in existing techniques. This then has the result that the method in accordance with the invention is more economical than existing techniques.

Contrary to existing techniques, the analysis of CO₂ reactivity using the procedure in accordance with the invention will be a study of a pre-oxidised sample, as the sample will already have been exposed to air in the air reactivity analysis. This is an advantageous aspect of the invention because the CO₂ reactivity measured on a pre-oxidised sample will be more in accordance with the real conditions in the electrolysis cells.

The method in accordance with the invention is characterised in that the air and CO₂ reactivity of a sample of a carbon product are decided from the same test, first by analysing the air reactivity and, when this analysis is complete, automatically analysing the CO₂ reactivity on the same sample. Finally the soot index is decided by collecting and weighing the soot dust from the sample.

Preferred features of this method is to analyse the air reactivity at 525°C and the CO₂ reactivity at 960°C.

The reaction between the CO₂ and the carbon is endothermic, whereas the air reaction, which is normal combustion, is exothermic. Both are strongly dependent on temperature. This phenomenon creates a dilemma in choosing the test temperature. On the one hand the test should take place at temperatures close to those found in the electrolysis cells in order not to miss out on the factors which are important for reactivity and sooting. It has, for example, been demonstrated that several elements and compounds can occur as accelerators and inhibitors in these reactions; however, the effect is dependent on temperature. On the other hand, this must be weighed against the possibilities for designing equipment and a practicable method for registering what happens. In this procedure, a compromise has been made by carrying out the CO₂ test at 960°C and the air test at 525°C. This is the most practical method even if these temperatures are somewhat lower than the temperature in the anode's wear surface, the top and shoulders respectively, of which they are to give a convincing representation.

The present invention also covers equipment for carrying out the above-mentioned method.

The equipment is characterised by a vertical tube furnace with an inlet for the introduction of gas, and a sample holder for the carbon product which is suspended freely from a weighing device and reaches down into the tube furnace; the sample holder is provided with one or more thermocouples for recording the temperature in the carbon product.

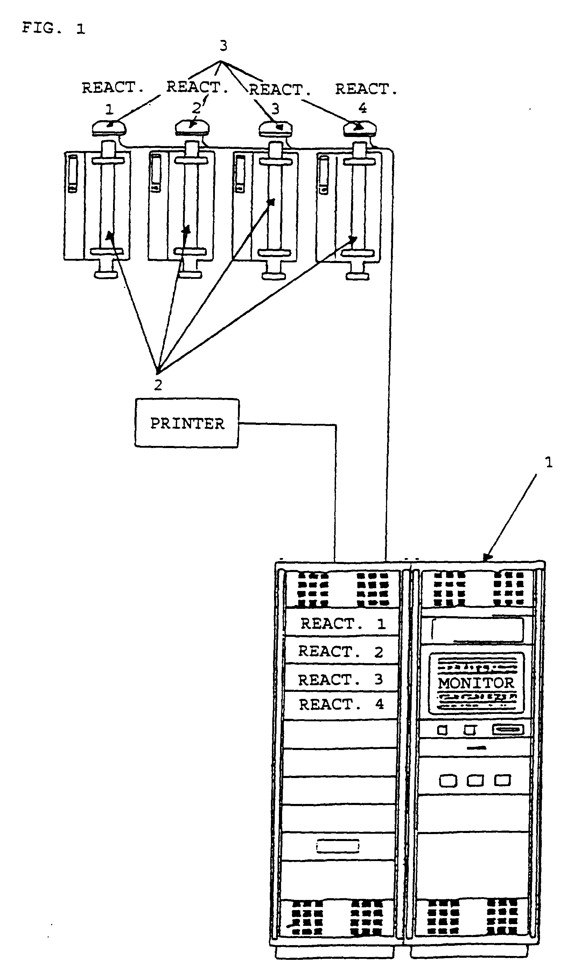

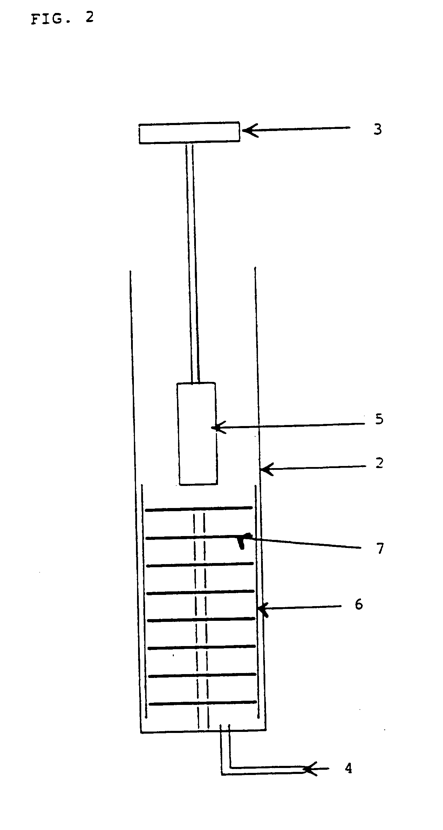

The equipment is shown in figures 1 and 2, in which the symbols 1-7 stand for:
1. Processing unit
2. Vertical tube furnace
3. Weighing device
4. Inlet for the introduction of gas
5. Sample holder
6. Radial radiation shield
7. Heating element

The method comprises the use of computer equipment for process control, data logging and calculation. In principle there is no limit to the number of samples which can be analysed in parallel by connecting a number of analysis devices to the computer equipment.

The loss of weight of the carbon sample due to gasification by air and carbon dioxide is measured continuously by the processing unit which is connected to the weighing device. The thermocouples are also connected to the processing unit so that the temperature in the sample can be recorded and checked. The temperature in the furnace and the temperature in the sample are regulated by the processing unit.

In the analysis of burned carbon core samples the sample piece has the form of a cylinder. The sample holder which is used in this instance comprises a flange, on which is fastened a pin, threaded at the lower end, a thermocouple and an endpiece with complementary threads which can be screwed onto the pin to fasten the sample piece which is provided with two holes which fit the pin and the thermocouple. A ceramic pipe is fastened at the top of the flange; at the other end the pipe is provided with a suspension device.

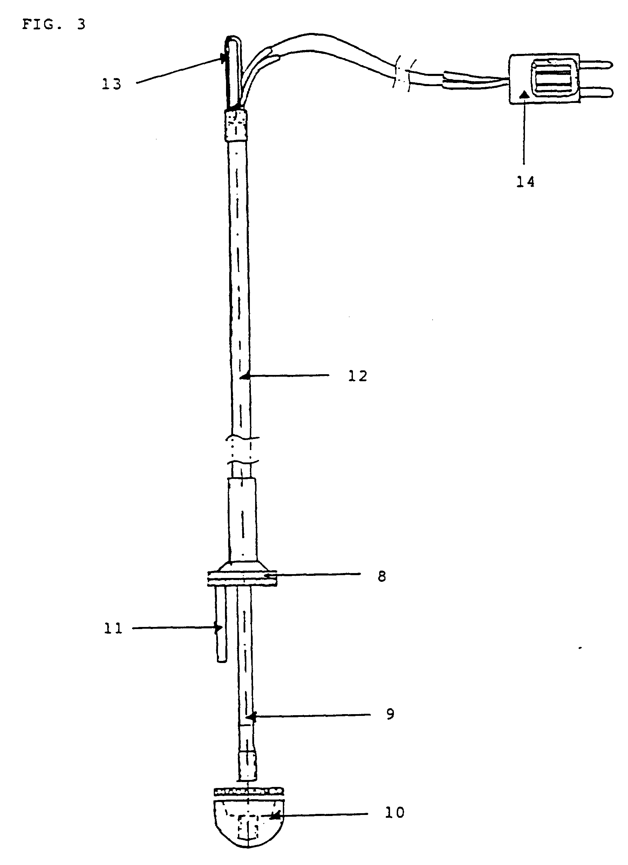

This sample holder is shown in figure 3 and the symbols 8-14 stand for:
8. Flange
9. Pin
10. Endpiece
11. Thermocouple
12. Ceramic pipe
13. Suspension device for weighing
14. Plug for thermocouple

Only the side surface of the cylinder-shaped sample piece takes part in the reaction. The end surfaces are not exposed because the sample holder's flange and endpiece cover them. The reactant gas flows in laminar flow up along the sample to create identical reaction conditions over the whole surface.

It is important that the temperature is kept constant over the whole reaction surface.

In the analysis of granular carbon products another sample holder is used. Here the sample holder itself is a thermocouple. This sample holder comprises a crucible which is sealed or perforated at the base, where the carbon granulate is placed; the crucible is provided with wires which are surrounded by ceramic pipes which are collected in a ceramic pipe, at the top of which there is a suspension device. The crucible and two of the wires are of platinum, whereas one of the wires is of platinum and rhodium. The temperature registration takes place at the point at which the wire of platinum and rhodium is fastened to the crucible.

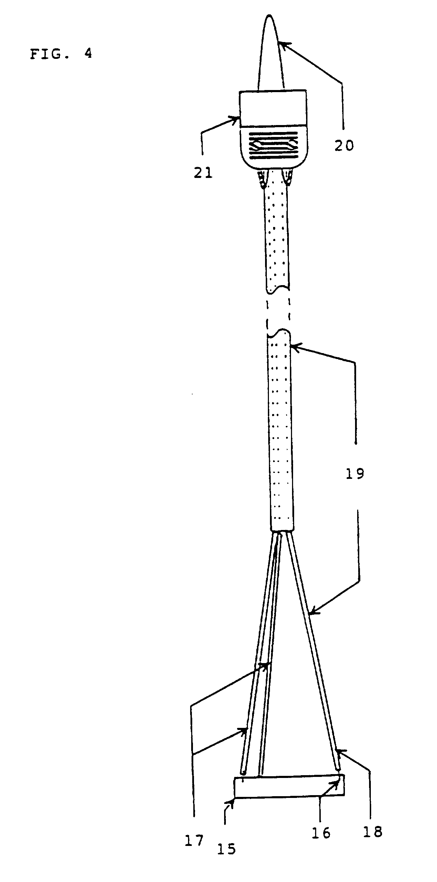

The sample holder which is used in the analysis of granular coke is shown in figure 4 in which the symbols 15-21 stand for:
15. Crucible (Pt)
16. Point at which temperature is registered
17. Wires (Pt)
18. Wire (Pt and Rh)
19. Ceramic pipe
20 Suspension device for weighing
21 Plug for thermocouple

Both of the sample holders described above are shaped in such a way that the thermocouple is in direct contact with the carbon product during the analysis. This causes the temperature recording to be very accurate.

The invention will be further explained in the following by means of examples.

The gas is introduced into tube furnace 2, which is made of gold, via an inlet 4 at the base of the tube furnace, and it is preheated to reaction temperature as it passes a radial radiation shield 6 inside the tube furnace on the way towards the carbon sample. The introduction of gas is regulated by the processing unit 1. Such generous amounts of gas are introduced that a further increase has no influence on the test result.

The analysis of the test result is carried out automatically by the processing unit 1 via dialogue boxes. The processing unit changes from the introduction of a gas to another automatically. During the heating of the sample inert atmosphere (N₂) is introduced. The processing unit automatically closes the N₂ valve and opens the air or CO₂ valve. When the reaction has been completed, the processing unit automatically switches back to N₂ and the sample is cooled down. The standard conditions during the analysis are:

| | |
|---|---|
| Heating time | 60 minutes |
| Reaction time | 180 minutes |
| Cooling time | 30 minutes |
| | |
| Reaction temperature in the CO₂ | 960°C |
| Reaction temperature in the air | 525°C |

The flow of gas through the furnace is 100 Nl/h of CO₂ and 200 Nl/h of air.

However, these reaction conditions may be easily changed by the operator.

The weighing system in the apparatus has a reproducibility of 1 mg. The weight is recorded continuously (every 20 seconds under standard conditions). The high number of measurements, the good reproducibility of the weighing system and the advanced temperature control which is within ± 1° of the desired temperature, ensure high precision results. The precision is better than ± 1 %.

The results of the analysis are calculated by the processing unit 1.

In an apparatus consisting of 8 tube furnaces, it is possible to analyse 8 carbon samples in the course of 4.5 hours. The time required to prepare a carbon sample for analysis is 10 minutes. As mentioned above, the processing unit controls the furnaces automatically. The time required for an operator to be able to prepare the samples, fasten the samples in the furnaces, remove the samples from the furnaces, collect the soot and read off the results for samples in 8 furnaces is a total of 100 minutes.

Samples of different carbon anode materials were analysed taking into account the air reactivity, the CO₂ reactivity and the soot index. Standard procedures as indicated above were used during the tests. When the air reactivity at 525°C had been completely analysed, the furnace temperature was automatically increased to 960°C for the analysis of the CO₂ reactivity.

For comparison, the anode samples were also analysed in two separate tests in accordance with existing techniques, for the CO₂ reactivity, the air reactivity and the soot index.

The results from the reactivity measurements when using the procedure according to the present invention and when using existing techniques are shown in table 1.

**Table 1.**

| Results from reactivity measurements. | | | | | | | | |
|---|---|---|---|---|---|---|---|---|
| | Test | | | Ref. test | | | Ref. test | |
| No. | Reac. CO₂ mg/cm²h | Soot index % | Reac. air mg/cm²h | Reac. CO₂ mg/cm²h | Soot index % | Reac. air mg/cm²h | Reac. CO₂ mg/cm²h | Soot index % |
| 1 | 26.2 | 12.3 | 20.4 | 12.4 | 4.2 | 27.1 | 12.5 | 5.5 |
| 2 | 22.6 | 9.8 | 18.3 | 12.4 | 4.7 | 18.4 | 12.0 | 6.9 |
| 3 | 19.1 | 7.6 | 17.0 | 13.5 | 5.3 | 18.8 | 13.1 | 6.0 |
| 4 | 21.3 | 23.7 | 20.4 | 13.5 | 12.7 | 24.5 | 12.2 | 7.9 |
| 5 | 21.8 | 11.7 | 18.4 | 15.0 | 4.1 | 19.1 | 14.2 | 7.4 |
| 6 | 33.4 | 8.4 | 19.3 | 24.1 | 5.9 | 21.7 | 22.0 | 4.3 |
| 7 | 29.9 | 9.9 | 21.7 | 19.7 | 10.0 | 20.6 | 21.3 | 10.5 |
| 8 | 44.2 | 10.8 | 23.3 | 25.1 | 6.0 | 24.0 | 22.0 | 5.3 |
| 9 | 44.8 | 11.1 | 24.4 | 28.7 | 6.3 | 25.7 | 21.2 | 6.1 |
| 10 | 40.8 | 7.5 | 21.4 | 23.0 | 3.2 | 26.9 | 20.8 | 5.1 |
| 11 | 53.3 | 23.9 | 31.4 | 30.6 | 10.5 | 37.1 | 29.6 | 13.1 |
| 12 | 50.9 | 19.1 | 30.4 | 31.6 | 10.1 | 24.9 | 31.9 | 8.7 |
| 13 | 49.3 | 24.2 | 34.7 | 34.6 | 11.5 | 33.1 | 29.7 | 11.1 |
| 14 | 49.5 | 22.8 | 35.5 | 30.5 | 12.4 | 33.8 | 30.6 | 12.8 |
| 15 | 51.3 | 43.3 | 47.1 | 35.3 | 28.0 | 46.0 | 30.1 | 28.5 |

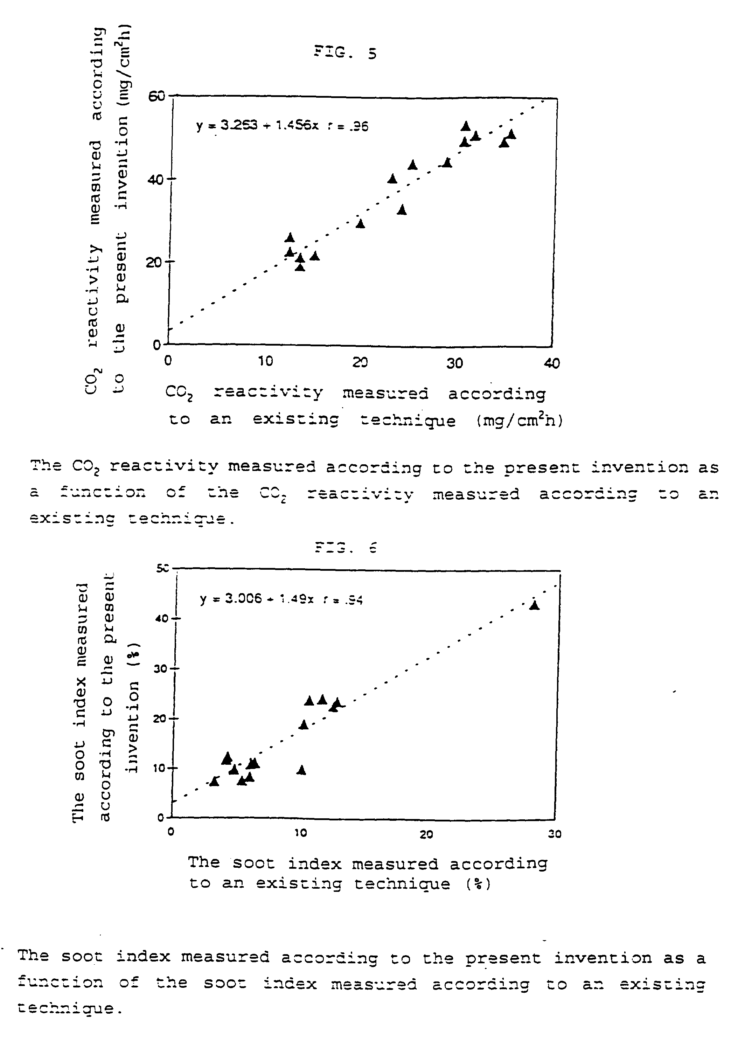

Figures 5 and 6 show the CO₂ reactivity and soot index analysed in accordance with the procedure in the present invention as a function of the reactivity measured by an existing technique. Figure 5 shows that there is good correspondence between the results for the reactivity analysed by means of the invention and an existing technique. The measurements in accordance with the present invention provide results in this case which are 1.5 times higher than the reactivity measurements from the existing technique. This is due to the fact that the carbon samples examined in accordance with the procedure in the present invention were pre-oxidised as they had already been investigated for air reactivity. The results achieved by using the method in accordance with the present invention for CO₂ reactivity and the soot index are therefore probably more closely related to the realistic conditions in an electrolysis cell.
The correlation coefficient is good (0.96).

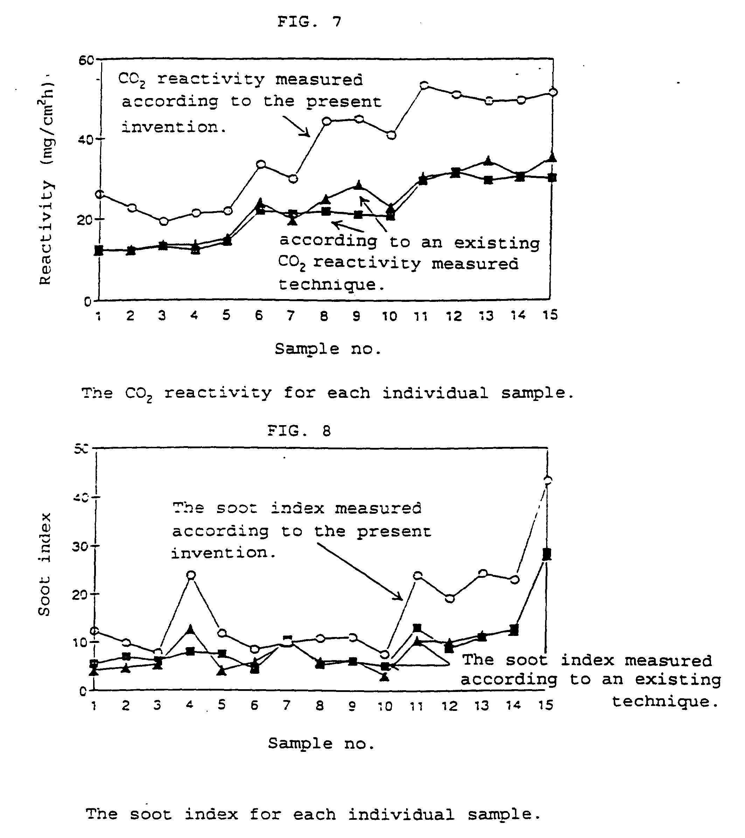

In figures 7 and 8 the results from the CO₂ reactivity and the soot index are plotted for each individual sample.

## Claims

1. A method for determining air reactivity, CO₂ reactivity and soot index of a carbon product, characterised by the following steps :
- first conducting an air reactivity analysis on a sample of said carbon product and thereby determining said air reactivity thereof;
- after completion of said air reactivity analysis, then conducting a CO₂ reactivity analysis on said air reacted sample of said carbon product and thereby determining said CO₂ reactivity thereof; and
- after completion of said CO₂ reactivity analysis, collecting and weighing soot dust from said sample resulting from said analyses and thereby determining said soot index of said carbon product.

2. Method according to claim 1, characterised in that the air reactivity is analysed at 525° and the CO₂ reactivity is analysed at 960°C.

3. Equipment for carrying out the method according to anyone of the claims 1 or 2,
comprising a furnace with an inlet for the introduction of gas, a weighing device and a sample holder for the carbon product, said sample holder being provided with one ore more thermocouples for registering the temperature in the carbon product,
characterised in that the furnace is a vertically arranged tube furnace (4), whereby the sample holder (5) is freely suspended from the weighing device (3) and reaches down into the tube furnace.

4. Equipment according to claim 3 for deciding the air reactivity, the CO₂ reactivity and
the soot index of a sample piece of carbon, characterised in that the sample holder (5) comprises a flange (8) on which a pin (9) is fastened which is threaded at its lower end, a thermocouple (11), and an endpiece (10) with complementary threads which can be screwed onto the pin to fasten the sample piece which is provided with two holes which fit the pin and the thermocouple; a ceramic pipe (12) is fastened at the top of the flange, provided with a suspension device (13) at its lower end.

5. Equipment according to claim 3, for deciding the air reactivity, the CO₂ reactivity and
the soot index of a granulated carbon product, characterised in that the thermocouple is the sample holder (5) itself comprising a crucible (15) which is sealed or perforated at the base, where the granular carbon is placed; the crucible is provided with wires (17, 18) which are surrounded by ceramic pipes (19) which are meet in one ceramic pipe, the top of which is provided with a suspension device (20).

6. Equipment according to claim 5, characterised in that the crucible (15) and the wires (17) consist of platinum and the wire (18) consists of platinum and rhodium and where temperature registration takes place at the point (16) at which the wire (18) is fastened to the crucible.

## Patentansprüche

1. Verfahrten zur Bestimmung der Reaktivität von Luft, der Reaktivität von CO₂ und des Rußindexes eines Kohlenstoffproduktes, gekennzeichnet durch die nachfolgenden Verfahrensstufen:
- vorerst die Durchführung einer Analyse der Reaktivität von Luft an einer Probe des Kohlenstoffproduktes und dadurch die Bestimmung der Reaktivität von Luft an der besagten Probe;
- alsdann, nach Abschluß der Analyse der Reaktivität von Luft, die Durchfuhrung einer Analyse der Reaktivität von CO₂ an der Probe des Kohlenstoffproduktes aus der Reaktivitätsbestimmung an Luft und dadurch die Bestimmung der Reaktivität von CO₂ an der besagten Probe; und
- nach Abschluß der Analyse der Reaktivität von CO₂, das Auffangen und das Abwiegen von Rußstaub aus der sich aus den besagten Analysen ergebenden Probe und dadurch die Bestimmung des Rußindexes des Kohlenstoffproduktes.

2. Verfahren gemäß Anspruch 1, dadurch gekennzeichnet, daß die Reaktivität von Luft bei 525°C analysiert wird und die Reaktivität von CO₂ bei 960°C analysiert wird.

3. Vorrichtung zur Durchführung des Verfahrens gemäß irgendeinem der Ansprüche 1 oder 2, welche einen Ofen umfaßt der einen Einlaß für die Einleitung von Gas, eine Wägevorrichtung sowie einen Probenhalter für das Kohlenstoffprodukt aufweist, wobei der Probenhalter mit einem oder mehreren Thermoelementen zur Registrierung der Temperatur in dem Kohlenstoffprodukt ausgestattet ist, dadurch gekennzeichnet, daß der Ofen ein vertikal angeordneter Röhrenofen (4) ist, wodurch der Probenhalter (5) frei von der Wägevorrichtung (3) herunterhängend aufgehängt ist und bis hinunter in den Röhrenofen reicht.

4. Vorrichtung gemäß Anspruch 3 zur Bestimmung der Reaktivität von Luft, der Reaktivität von CO₂ und des Rußindexes eines Probestückes aus Kohlenstoff, dadurch gekennzeichnet, daß der Probenhalter (5) ausgestattet ist mit einem Flansch (8), an welchem ein Bolzen (9) befestigt ist der an seinem unteren Ende mit einem Gewinde versehen ist, einem Thermoelement (11) und einem Endstück (10) mit komplementären Gewinden die auf den Bolzen aufgeschraubt werden können zwecks Befestigung des Probestückes das mit zwei auf den Bolzen und auf das Thermoelement passenden Bohrungen versehen ist; ein Rohr aus Keramik (12) ist an dem oberen Ende des Flansches befestigt und an seinem Fußende mit einer Aufhängevorrichtung (13) ausgestattet.

5. Vorrichtung gemäß Anspruch 3 zur Bestimmung der Reaktivität von Luft, der Reaktivität von CO₂ und des Rußindexes eines granulierten Kohlenstoffproduktes, dadurch gekennzeichnet, daß das Thermoelement den Probenhalter (5) selbst darstellt und einen Tiegel (15) aufweist der an seiner Basis dort abgedichtet oder durchlöchert ist wo der granulierte Kohlenstoff hingebracht wird; der Tiegel ist mit Drähten (17, 18) ausgestattet die von keramischen Rohren (19) umgeben sind und sich in einem keramischen Rohr zusammenfinden dessen oberes Ende mit einer Aufhängevorrichtung (20) ausgestattet ist.

6. Vorrichtung gemäß Anspruch 5, dadurch gekennzeichnet, daß der Tiegel (15) und die Drähte (17) aus Platin bestehen und der Draht (18) aus Platin und Rhodium besteht und daß dabei die Aufzeichnung der Temperatur an dem Punkt (16) stattfindet wo der Draht (18) an dem Tiegel befestigt ist.

## Revendications

1. Procédé pour la détermination de la réactivité d'air, de la réactivité de CO₂ ainsi que de l'indice de suie d'un produit de carbone, caractérisé par les étapes suivantes:
- d'abord l'exécution d'une analyse de la réactivité d'air sur un échantillon dudit produit de carbone, ainsi que la détermination de ladite réactivité d'air par cette voie;
- puis, à la suite de l'achèvement de ladite analyse de la réactivité d'air, l'exécution d'une analyse de la réactivité de CO₂ sur ledit échantillon de réaction dudit produit de carbone avec l'air, ainsi que la détermination de ladite réactivité de CO₂ par cette voie; et
- à la suite de l'achèvement de ladite analyse de la réactivité de CO₂, le captage et la pesée de la poussière de suie hors dudit échantillon résultant desdites analyses, ainsi que la détermination dudit indice de suie dudit produit de carbone par cette voie.

2. Procédé suivant la revendication 1, caractérisé en ce que la réactivité d'air est analysée à 525°C et que la réactivité de CO₂ est analysée à 960°C.

3. Equipement pour l'exécution du procédé suivant l'une quelconque des revendications 1 ou 2, comprenant un four avec une admission pour l'introduction de gaz, un dispositif de pesée ainsi qu'un porte-échantillon pour le produit de carbone, ledit porte-échantillon étant pourvu d'un ou de plusieurs thermocouples pour enregistrer la température dans le produit de carbone, caractérisé en ce que le four est un four tubulaire (4) disposé verticalement, moyen par suite duquel le porte-échantillon (5) est suspendu librement à partir du dispositif de pesée (3) et descend vers le bas dans le four tubulaire.

4. Equipement suivant la revendication 3 pour la détermination de la réactivité d'air, de la réactivité de CO₂ et de l'indice de suie d'un fragment d'échantillon de carbone, caractérisé en ce que le porte-échantillon (5) comporte une aile (8) à laquelle est fixée une broche (9) qui est filetée à son extrémité inférieure, un thermocouple (11) ainsi qu'une pièce terminale (10) avec des filetages complémentaires qui peuvent être visés sur la broche pour fixer le fragment d'échantillon qui est pourvu de deux alésages qui s'emboîtent sur la broche ainsi que sur le thermocouple; un tube en céramique (12) est fixé sur la partie supérieure de l'aile et il est pourvu d'un dispositif de suspension (13) à son extrémité d'aval.

5. Equipement suivant la revendication 3 pour la détermination de la réactivité d'air, de la réactivité de CO₂ et de l'indice de suie d'un produit de carbone granulé, caractérisé en ce que le thermocouple est lui-même le porte-échantillon (5) et comporte un creuset (15) qui est scellé ou perforé à sa base, là où le carbone granulé est placé; le creuset est équipé de fils (17, 18) qui sont entourés par des tubes en céramique (19) et qui se rencontrent dans un seul tube en céramique dont la partie supérieure est pourvue d'un dispositif de suspension (20).

6. Equipement suivant la revendication 5, caractérisé en ce que le creuset (15) et les fils (17) sont constitués de platine et que le fil (18) est constitué de platine et de rhodium alors que l'enregistrement de la température se déroule à l'endroit (16) où le fil (18) est fixé au creuset.
